# EUROPÄISCHE PATENTSCHRIFT

(11) **EP 2 148 705 B1**
(45) Veröffentlichungstag und Bekanntmachung des Hinweises auf die Patenterteilung: **16.07.2014**
(21) Anmeldenummer: 08748792.2
(22) Anmeldetag: 13.05.2008
(51) Int. Cl.: A61L 27/30, A61L 27/54

(54) **IMPLANTAT MIT EINER BIOTOXISCHEN BESCHICHTUNG UND VERFAHREN ZU DESSEN HERSTELLUNG**
IMPLANT COMPRISING A BIOTOXIC COATING, AND METHOD FOR THE PRODUCTION THEREOF
IMPLANT POURVU D'UN REVÊTEMENT BIOTOXIQUE ET PROCÉDÉ DE PRODUCTION DE CET IMPLANT

(30) Priorität: 26.05.2007 DE 102007023294
(43) Veröffentlichungstag der Anmeldung: 03.02.2010
(73) Patentinhaber: Fachhochschule Kiel, 24149 Kiel (DE)
(72) Erfinder: ES-SOUNI, Mohammed, 24247 Mielkendorf (DE)
(74) Vertreter: Hansen, Jochen
(86) Internationale Anmeldenummer: PCT/DE2008/000814
(87) Internationale Veröffentlichungsnummer: WO 2008/145088

(56) Entgegenhaltungen:
- EP-A- 1 291 033
- WO-A-2004/026346

## Beschreibung

Die Erfindung betrifft ein Implantat mit einer biotoxischen Beschichtung. Insbesondere betrifft die Erfindung ein Implantat mit einer Silberionen enthaltenden Titanoxidbeschichtung, wobei die Silberionen durch thermische Sinterung eines Precursor-Materials in die Beschichtung eingebettet sind. Die Erfindung betrifft weiterhin ein Verfahren Verfahren zum Herstellen einer Implantatbeschichtung mit definierter Toxizität.

Zu den Problemen der Implantatmedizin zählt bekanntlich die Abstoßungsreaktion. Um die körpereigene Abwehr zu überlisten, werden Implantate (z.B. Herzschrittmacher) heute mit biokompatiblen Beschichtungen versehen, auf denen ein günstiges Milieu für die Proliferation von Gewebezellen vorherrscht. Dies begünstigt das Einwachsen des Implantats, was zusätzlich noch medikamentös unterstützt werden kann.

Implantate mit Medikament-freisetzender Beschichtung ("Drug-Eluting") sind mit vielfältigen Zielsetzungen in der Fach- und Patentliteratur, so z.B. der WO 2004/026346 A, vorgeschlagen worden und existieren bereits teilweise auf dem Markt. Dies betrifft ganz besonders Vaskularprothesen, so genannte Stents.

Bei den Stents ist das Problem bekannt, dass es auch zu Gewebebildung und/oder Zellanhaftung auf der Innenseite des Stents kommen kann, was dem Zweck der Stent-Implantation entgegenwirkt und lebensbedrohliche Folgen für den Patienten haben kann, denn dies kann zu Entzündungen oder zum Zuwachsen des Stent-Inneren führen. Dieses Problem ist besonders bei Stents, welche über Gefäßbifurkationen platziert werden müssen, akut, da dann die Nebengefäße im Falle einer In-Stent-Restenose nicht mehr mit Blut versorgt werden können. Wünschenswert ist daher eine biotoxische Beschichtung des Stent-Inneren, die das Anhaften von Zellen durch sehr langsame Vergiftung verhindert. Andere, vorbeibewegte Zellen (z.B. rote Blutkörperchen im Blutfluß) dürfen natürlich nicht beeinträchtigt sein.

Es wurde vorgeschlagen, durch örtliche Medikamentenfreisetzung diese Inflammations- und Koagulationsreaktionen in Stents zu verhindern. Randomisierte Studien haben jedoch gezeigt, dass im Vergleich zu Kontrollgruppen (unbeschichtete Stents) das Ergebnis nicht signifikant unterschiedlich war [z.B. Antonio Colombo, Jeffrey W. Moses, Marie Claude Morice et al., "Randomized Study to evaluate Sirolimus-Eluting Stents at Coronary Bifurcation Lesions", Circulation 2004; 109: 1244-1249].

An anderer Stelle wird über (galvanische) Goldbeschichtung von koronaren Stents berichtet [Edelmann ER et al., "Gold-coated NIR stents in porcine coronary arteries", Circulation 2001; 103:429-434]. Die in vivo Tests ergaben keinerlei Verbesserung im Tierversuch gegenüber der Kontrollgruppe. Es wurde sogar eine verstärkte Inflammation und In-Stent-Restenose bei goldbeschichteten Stents verzeichnet [Kastrati A et al., "Increased risk of restenosis after placement of gold-coated stents: results of a randomized trial comparing gold-coated with uncoated steel stents in patients with coronary artery disease", Circulation 2004; 101: 2478-2483].

Weitere Studien berichten über Kohlenstoffbeschichtungen (DLC) ohne Angaben über einen Unterschied zu Kontrollgruppen. im Tierversuch zu machen [Galloni M, Prunotto M et al., "Carbon-coated stents implanted in porcine iliac and renal arteries: histological and histomorphic study", J. Vasc. Radiol. 2003; 14: 1053-1061; Ralf Max Beck, "Untersuchung von Oberflächenbeschichtungen bei Gefäßstützen zur Reduktion von Restenosen", Doktorarbeit Uni Tübingen 2001]. In einer Übersichtstudie wurden SiC-, DLC- und medikamentenbeschichtete Stents in Bezug auf ihre Wirkung zur Reduzierung der In-Stent Restenoserate evaluiert [Babapulle MN, Eisenberg MJ., "Coated stents for the prevention of restenosis: Part II", Circulation 2002; 106: 2859-2866]. Es hat sich gezeigt, dass alle Beschichtungen nur einen marginalen Einfluss auf die Restenoserate haben.

Eine Stentbeschichtung darf durch den fortwährenden Vorbeifluß von Blut auch nicht beschädigt oder gar allmählich abgetragen werden. Überdies sollte sie auf der Stent-Außenseite deutlich verringerte Biotoxizität zeigen, um eben das Einwachsen des Implantats zu fördern. Es wurde deshalb schon vorgeschlagen, Bereiche unterschiedlicher Biokompatibilität auf demselben Implantat einzurichten.

Die Druckschriften WO 01/45862 A1 und US 2004/0126596 A1 offenbaren die Möglichkeit einer Plasma-Behandlung von Implantat-Oberflächen mit dem Ziel, die Proliferation bestimmter Zellen an diesen zu unterbinden oder eine Apoptose herbeizuführen. Insbesondere wird aber auch vorgeschlagen, geeignete Monomere via Plasma-Deposition abzuscheiden, um biokompatible Polymerschichten auf den Substraten zu bilden. Da Plasmaverfahren grundsätzlich nur unter Vakuum oder straten zu bilden. Da Plasmaverfahren grundsätzlich nur unter Vakuum oder Schutzgasatmosphäre durchführbar sind, ist das erforderliche Equipment und Prozess-Know-How nur bei den Implantat-Herstellern zu erwarten.

Als eine Komplikation bei medizinischen Eingriffen, bei denen ein Stent implantiert wird, kann es sich auch erweisen, dass etwa ein Stent in der Nähe einer Gefäßverzweigung so eingesetzt werden muss, dass seine Außenseite nicht völlig mit der Gefäßwand abschließt. Ist diese Außenseite nun durch Vorbehandlung bioverträglich ausgebildet worden, so begünstigt dies wiederum die Zellhaftung an ungewollter Stelle, nämlich im Blutstrom im Bereich der Verzweigung.

Wünschenswert wären somit Implantat-Beschichtungen, die sich im Nachgang der Fertigung und des Verkaufs mit einfachen Mitteln von medizinischem Personal strukturiert hinsichtlich Biotoxizität modifizieren ließen. Dies sollte durch einfachen Energieeintrag (z.B. Licht, Wärme, elektrische Entladung, o. ä.) geschehen können, wobei ein einfacher, handgehaltener Applikator ein mögliches Werkzeug zur Funktionalisierung sein könnte. Als Vorbild sei auf UV-Lampen in der zahnärztlichen Praxis verwiesen, mit denen etwa Zahnfüllungen und dergleichen beschleunigt gehärtet werden können.

Von dem Erfinder wurde in einer anderen, nicht-vorveröffentlichten Anmeldung ein Verfahren zur Erzeugung von Substratbeschichtungen für die Oberflächen-verstärkte ("surface enhanced") Raman-Spektroskopie (SERS) vorgeschlagen, bei dem mittels eines Sol-Gel-Verfahrens zunächst eine Silber-Ionen enthaltende Titanoxid-Schicht erzeugt wird, die durch nachträgliche Beleuchtung mit - überwiegend sichtbarem-Licht unter gleichzeitiger Erwärmung Silber-Nanopartikel auf der Oberfläche ausbildet.

Aus der Patentschrift US 6 482 444 B1 ist zwar bekannt, dass Sol-Gel-Beschichtungen mit silberhaltigen Glaskeramiken antibakterielle Wirkung zeigen und vor allem bei Knochen- und Gelenkimplantaten die Gewebeanbindung verbessern und Entzündungen hemmen. Silber ist allerdings ein bekanntes Zellgift und für bioverträgliche Implantate a priori nicht zu empfehlen.

Der Erfindung, liegt nun die Aufgabe zugrunde, eine robuste Implantat-Beschichtung zu schaffen, die unmittelbar nach ihrer Herstellung hohe Biotoxizität aufweist, welche durch nachträgliche Bearbeitung in einfacher Weise modifiziert, d.h. insbesondere auf vorbestimmten Teilflächen vermindert, werden kann.

Diese Aufgabe wird mehrfach gelöst, nämlich durch das Verfahren zum Herstellen einer Implantatbeschichtung mit definierter Toxizität mit den Merkmalen von Anspruch 1.

Die Aufgabe wird auch durch Bereitstellung eines Implantats mit den Merkmalen von Anspruch 10 gelöst.

Die Unteransprüche geben jeweils vorteilhafte Ausgestaltungen der Erfindung wieder.

Erfindungsgemäß wird eine Silber-Ionen enthaltende Titanoxidschicht auf dem Implantat hergestellt, welche - wie sich zeigt - tatsächlich biotoxisch wirksam ist. Weiterhin erfindungsgemäß wird die hergestellte Implantat-Beschichtung zu einem späteren Zeitpunkt, insbesondere vor oder während eines medizinischen Eingriffs an einem Patienten, wenigstens auf Teilflächen mittels Beleuchtung unter gleichzeitiger Erwärmung nachbearbeitet, so dass auf diesen Teilflächen die Biotoxizität deutlich vermindert wird.

Es ist vorgesehen, dass die Teilflächen mit verminderter Biotoxizität vom behandelnden Arzt so festgelegt werden können, dass sie den Teilflächen des Implantats entsprechen, an denen ein Anwachsen des umgebenden Gewebes medizinisch gewünscht ist. Ihm steht dazu vorteilhafterweise die Kenntnis des konkreten Patienten und der Details des Eingriffs zur Verfügung. Dies war zum Zeitpunkt der Herstellung des Implantats bzw. der Herstellung der Beschichtung natürlich nicht bekannt.

Die Erfindung wird nachfolgend näher erläutert; dabei zeigt:
- Fig. 1: einen Vergleich der bakteriziden Wirkung belichteter (ir) und nichtbelichteter (nir) SER-Schichten auf in Kontakt befindliche Suspensionskulturen (Med: Medium; Mir: Medium mit belichteter SER-Schicht; Mnir: Medium mit unbelichteter SER-Schicht, NK: Bakterienkultur ohne Schicht)
- Fig. 2: Ergebnisse einer Zellproliferationsuntersuchung mittels BrdU-Test (Abkürzungen analog zu Fig. 1);
- Fig. 3: eine Zellkultur an der Grenze zwischen belichteten und unbelichteten Bereichen der erfindungsgemäßen SER-Schicht auf einem Implantatstahl.

Die erfindungsgemäße Implantat-Beschichtung wird folgendermaßen gebildet:
Angesetzt an der Lehre der WO 2006/060734 A2 wird auch hier von einem zusätzliche Edelmetall-Ionen enthaltenden Precursor zur Herstellung eines Metalloxid-Films über ein Sol-Gel-Verfahren ausgegangen wird. Der Precursor muss allerdings für die Realisierung der Erfindung notwendig Titan enthalten, um eine Titanoxid-Matrix, vorzugsweise TiO₂, bilden zu können.

Als Edelmetall-Ionen werden ganz besonders bevorzugt Silber-Ionen verwendet. Andere Edelmetalle sollen zwar nicht ausgeschlossen werden, doch liegen hierfür noch keine Untersuchungen vor.

Die Erfindung bildet das bekannte Verfahren nun in unerwarteter Weise fort, indem der Silber-Ionen enthaltende Titan-Precursor zunächst auf ein wärmebeständiges Substrat (z.B. Glas, Halbleiter, Metall) mit einem Sol-Gel-Verfahren (insbesondere Schleudern, Sprühen, Tauchen) aufgebracht und dort umgehend unter Lichtausschluss pyrolysiert und gesintert wird. Nach der Wärmebehandlung ist die Schicht trocken und hart und weitgehend resistent gegenüber chemischem Angriff. Sie weist praktisch keine Silber-Nanopartikel auf der Oberfläche auf und eignet sich somit nicht als SER-Substrat. Eine weitere Bearbeitung nach der Lehre der WO 2006/060734 A2 ist auch nicht geeignet, dies zu verbessern. Die Schicht ist bei Aufbewahrung unter Lichtausschluss sehr gut lagerfähig.

Wird die silberhaltige Titanoxid-Schicht nunmehr unter ausreichender Erwärmung intensiv beleuchtet, so werden in der Matrix Elektron-Loch-Paare erzeugt gemäß

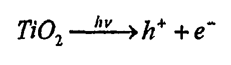

Anwesende Silber-Ionen tendieren stark zur Aufnahme der freigesetzten Elektronen.

*Ag*⁺ +1*e*⁻ → *Ag - Metall*

Es bilden sich kleinste Silberpartikel, die in der zugleich erwärmten Matrix eine gewisse Beweglichkeit aufweisen. Sie können sich durch Diffusion auch zu größeren Partikeln verbinden. Silber in unmittelbarer Nähe der Schichtoberfläche tritt durch diese hindurch und bildet Silber-Nanopartikel auf der Oberfläche. Kühlt das nunmehr entstandene Silber-Titanoxid-Nanokomposit wieder auf Umgebungstemperatur ab, wird die Silberpartikel-Verteilung praktisch "eingefroren". Die mit Licht und Wärme behandelte, ursprünglich SER-inaktive Schicht eignet sich dann als SER-Substrat und zeigt dabei einen Verstärkungsfaktor, der mit kommerziell erhältlichen Substraten konkurrieren kann.

Zunächst wird eine TiO₂-Ag Precursorlösung (Sol) hergestellt. Der Silberanteil soll zwischen 10% und 60% Massenanteil bezogen auf die Gesamtmasse der (nach Pyrolyse und Sintern) getrockneten Schicht betragen. Vorzugsweise wird der Ag-Massenanteil zwischen 30% und 60 % eingerichtet. Im nachfolgenden Beispiel beträgt er etwa 50 %, was als besonders vorteilhaft anzusehen ist.

Für die Herstellung von 100 ml einer ca. 0,6 molaren Lösung werden zuerst 10 ml 2-Methoxyethanol und Acetylaceton in einem Becherglas vorgelegt. Dann wird das Ti-isopropoxid zugegeben, wonach man 30 Minuten rühren lässt. Als eine zweite Lösung werden 10 ml 2-Methoxyethanol mit Wasser gemischt. Nach 30-minütigem Rühren wird die wasserhaltige Lösung zu dem Ti-Acetylaceton-Komplex gegeben. Erneut lässt man 30 Minuten rühren. Für die Silberlösung werden 10 ml 2-Methoxyethanol in einem Becherglas vorgelegt und AgNO₃ und Pyridin (als Stabilisator) dazugegeben. Diesen Komplex muss man ebenfalls 30 Minuten rühren lassen. Danach kann die Silberlösung zu der stabilisierten und hydrolysierten Titan-Lösung zugegeben werden. Nach erneutem 30-minütigem Rühren wird zu der Lösung 2 g Polyethylenglycol 400 zugegeben, mit 2-Methoxyethanol auf 100 ml aufgefüllt und anschließend gefiltert. Das Polyethylenglycol dient der rissfreien Schichtausbildung. Die Bereiche der Einwaagen, d.h. der Molangaben sind der folgenden Tabelle zu entnehmen.

| Produkt | Edukt | **Mol** | in g beispielhaft |
|---|---|---|---|
| TiO₂-50%Ag | TiIVPropoxid Ti(OCHMet2)4 | **0,03-0,05** | 9,83 |
| | AgNO3 | **0,01-0,03** | 4,33 |
| | Methoxyethanol | **0,5-0,7** | 52,23 |
| | H2O | **0,05-0,2** | 2,48 |
| | Acetylaceton | **0,01-0,03** | 1,73 |
| Pyridin | Pyridin | **0,1-0,5** | 30,26 |
| | PEG400 | -- | 2,00 |

Ti-iso. = Ti-isopropoxid
Hacac = Acetylaceton
Verwendete Stöchiometrien:
- Ti-isoprop. : Hacac : H₂O =1 : 0,5 : 4 (mol)
- AgNO₃ : Pyridin = 1 : 15 (mol)

Die TiO₂-Ag-Schichten werden durch Tauchbeschichten hergestellt. Als Träger dient beispielhaft Implantat-Stahl. Das Pyrolysieren der Schichten erfolgt dann bei 250 °C. Die Endbehandlungstemperatur (Sinterschritt) liegt zwischen 450 und 550 °C. Die Dicke der Schichten kann zwischen 50 und 1000 nm betragen. Bei der thermischen Behandlung muss unbedingt darauf geachtet werden, dass diese unter Ausschluss von Licht (UV bis Ende VIS) durchgeführt wird. Nur so wird jegliche unkontrollierte Silber-Ausscheidung/Reduktion vermieden.

Nach der Herstellung können die Substrate sofort belichtet oder ggf. auch im Dunkeln eingelagert werden. Die Belichtung kann mit praktisch allen herkömmlichen Lampen erfolgen, die Licht im Spektralbereich von 280 bis 800 nm emittieren. Die TiO₂-Matrix absorbiert sehr gut im gesamten sichtbaren Bereich, weshalb TiO₂ auch als Solarabsorber bekannt ist. Sofern die Lampe dabei zugleich Wärme abgibt, die die Schicht aufheizt, kann die SER-Aktivierung durch Bildung der Silberpartikel auf der Schichtoberfläche bereits in Gang gesetzt werden. Alternativ können Lichtquellen niedrigerer Leistung (z.B. Laser- oder Lumineszenzdioden) in Kombination mit einer Wärmequelle, welche eine Temperatur von bis zu 250 °C erlaubt, verwendet werden.

Die Einstellung der Partikelgröße und Verteilung erfolgt durch das Zusammenwirken von Belichtung und Wärme. Die Belichtung führt zur Reduktion des Silbers von Silberoxid in elementares Silber, die Wärme zur Vergröberung der Teilchen durch Diffusion.

Die Wärmezufuhr sollte zu diesem Zweck so eingerichtet werden, dass die Schicht wenigstens Temperaturen oberhalb von 80 °C aufweist. Zu bevorzugen sind Temperaturen zwischen etwa 150 °C und 250 °C. Es hat sich indes als nicht vorteilhaft erwiesen, Temperaturen oberhalb von 250 °C zu verwenden, weil dann die Beweglichkeit der Silberpartikel zu groß würde. Es könnten sich recht ungleichmäßige Partikelverteilungen auf der Schichtoberfläche einstellen, die die SER-Aktivität beeinträchtigen.

Zusammengefasst weist das die Schicht folgende Vorteile gegenüber dem Stand der Technik auf:
- Es kommen nur industrielle Standardprozesse (Sol-Gel-Beschichtung, Pyrolyse, Maskierung, Belichtung) zum Einsatz, die mit entsprechend hoher Geschwindigkeit durchführbar sind.
- Titanoxid-Schichten kommen vielfach zum Einsatz und sind als chemisch stabil bekannt. Entsorgungskanäle für gebrauchte SER-Substrate sind somit auch bereits vorhanden.
- Abgesehen vom unvermeidlichen Ausbrennen der Organik bei der Pyrolyse werden keine weiteren Chemikalien benutzt und freigesetzt, d.h. es entsteht kein neues Entsorgungsproblem.
- Die Reproduzierbarkeit der SER-Substrate ist - wie immer - eine Frage der präzisen Prozesskontrolle. Die hier eingesetzten Prozesse werden von der Industrie ausnahmslos beherrscht und erfordern keine neuen Entwicklungen.

Die angestrebte abriebfeste biotoxische Beschichtung, mit einer Silber-Ionen enthaltenden Titanoxidschicht kennzeichnet sich dadurch aus, dass die Silberionen durch thermische Sinterung eines Precursor-Materials unter Lichtausschluss unreduziert auf Interkalationsplätzen in der Matrix eingebettet und durch Lichteinstrahlung reduzierbar sind.

Die eingebetteten Silberionen können durch Beleuchtung mit UV-Licht unter Umwandlung in weniger biotoxische Silber-Nanopartikel zur Freisetzung von Elektron-Loch-Paare in der Titanoxidmatrix vorliegen, so daß Schichten erlangbar sind, in denen vorbestimmte Bereiche durch UV-Beleuchtung im mehrminütigen Umfang in ihrer Biotoxizität gegenüber unbeleuchteten, noch Silber-Ionen enthaltenden Titanoxidschicht stark dadurch vermindert ist, daß die Ionen durch Diffusion in kleine metallische Silberpartikel umgewandelt sind.

Für eine Beschichtung wird also ein Silber-Ionen enthaltender Titan-Precursor zunächst mit einem Sol-Gel-Verfahren (insbesondere Schleudern, Sprühen, Tauchen) auf die Implantat-Oberfläche gebracht und dort umgehend unter Lichtausschluss pyrolysiert und gesintert. Für das Implantat-Material wird dabei vorausgesetzt, dass es gegenüber den Temperaturen zur Wärmebehandlung (bis ca. 600 °C) beständig ist, z.B. Implantat-Stahl. Nach der Wärmebehandlung ist die entstandene Titanoxid-Schicht mit enthaltenen Silber-Ionen trocken und hart und weitgehend resistent gegenüber chemischem Angriff. Sie ist robust gegen Abrieb und bedeckt vorzugsweise die gesamte Oberfläche des Implantats.

Das Implantat mit erfindungsgemäßer SER-Schicht ist nachweislich biotoxisch, wie weiter unten ausgeführt wird. Die SER-Schicht bewahrt ihre Eigenschaften auch über lange Zeit, wenn das Implantat unter Lichtausschluss (lichtdicht verpackt) aufbewahrt wird.

Wird die silberhaltige Titanoxid-Schicht beleuchtet, so werden in der Matrix Elektron-Loch-Paare erzeugt und anwesende Silber-Ionen tendieren stark zur Aufnahme der freigesetzten Elektronen, wobei sich kleinste Silberpartikel bilden, die durch Festkörperdiffusion - also bevorzugt bei Erwärmung - zu größeren Teilchen anwachsen.

Kühlt das nunmehr entstandene Silber-Titanoxid-Nanokomposit wieder auf Umgebungstemperatur ab, wird die Silberpartikel-Verteilung praktisch "eingefroren". Die mit Licht und Wärme behandelte, ursprünglich biotoxische SER-Schicht zeigt nach dieser Behandlung eine deutlich verringerte Biotoxizität.

Ursache der Biotoxizität der zuerst hergestellten Schicht dürfte die Fähigkeit zur Abgabe von Silber-Ionen an etwaig mit der Schicht in Kontakt befindliche Zellen (oder auch Bakterien) sein. Diese Zellgiftabgabe verhindert die Proliferation. Nach der Beleuchtung liegt ein Großteil des Silbers in reduzierter, metallischer Form als Nanopartikel vor. Diese Partikel befinden sich überdies größtenteils nicht an der Oberfläche, sondern liegen weiterhin eingebettet in der Titanoxid-Matrix. Die Fähigkeit der SER-Schicht zur Abgabe von Silber-Ionen wird somit sehr stark reduziert, und die Biotoxizität nimmt stark ab.

Eine mit 9,8 g (d.h. 0,035 mol) TiIVPropoxid-Edukt, 4,3 g AgNO₃ hergestellte Schicht (siehe Tabelle) kann zum Beipiel nach der Herstellung sofort belichtet oder ggf. auch im Dunkeln eingelagert werden. Die Belichtung kann mit praktisch allen herkömmlichen Lampen erfolgen, die Licht im Spektralbereich von 280 bis 800 nm emittieren. Die TiO₂-Ag-Schicht absorbiert sehr gut im gesamten sichtbaren Bereich, weshalb TiO₂ auch als Solarabsorber bekannt ist.

Bevorzugt ist hier allerdings die Verwendung einer UV-Lampe mit Wellenlängen im Bereich von 250 bis 400 nm (z.B. Leistung 100 W). Die SER-Schicht sollte auf Temperaturen um 80 °C erwärmt werden, was bereits durch die Bestrahlung der UV-Lampe geschehen kann. Typische Bearbeitungsdauern liegen vorzugsweise um 20 Minuten.

Die Einstellung der Partikelgröße und Verteilung erfolgt durch das Zusammenwirken von Belichtung und Wärme. Die Belichtung führt zur Reduktion des Silbers von Silberoxid in elementares Silber, die Wärme zur Vergröberung der Teilchen durch Diffusion.

Auf den erzeugten SER-Schichten werden Biotoxizitäts-Tests durchgeführt.

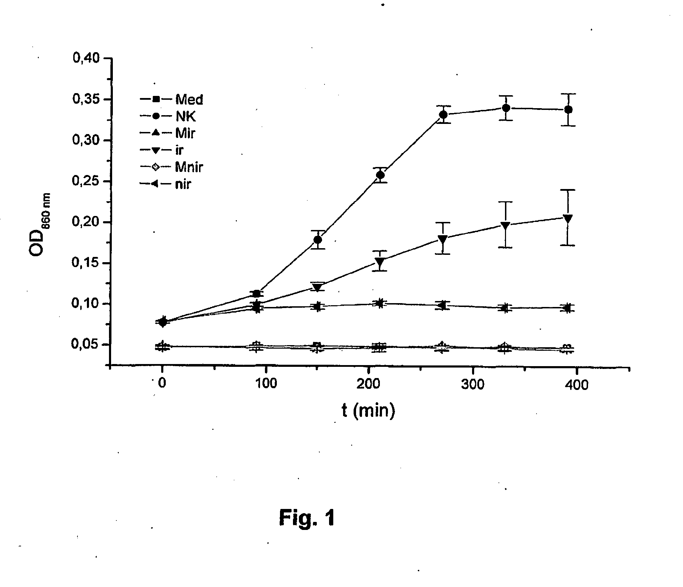

Die bakterizide Wirkung von belichteten und unbelichteten SER-Schichten wird an einer alpha-hämolysierenden Streptokokken-Mischkultur (apathogen), gewonnen von dem Rachenabstrich eines gesunden Donors, getestet. Dazu wird das Bakterienwachstum bei 38 °C über eine Trübungsmessung photometrisch bei 860 nm ermittelt. Das Ergebnis ist in Fig. 1 dargestellt. Aufgetragen ist die optische Dichte (OD) der Bakterienkultur als Funktion der Zeit. Die Kultur in Abwesenheit einer SER-Schicht (NK) zeigt die stärkste Trübung, also das maximale Wachstum. Die Kurven geringster Trübung (Med, Mir, Mnir) weisen keine Bakterienkultur auf, sondern nur das Suspensionsmedium ohne oder mit SER-Schicht (beleuchtet, nicht beleuchtet) als Referenz. Die maßgeblichen Messkurven (ir, nir) beschreiben das Verhalten des Bakterienwachstums auf der beleuchteten ("irradiated") und der nicht beleuchteten ("non-irradiated") SER-Schicht, respektive. Dabei zeigt sich, dass die optische Dichte für die Kultur auf der beleuchteten SER-Schicht etwa halbiert wird gegenüber der reinen Kultur ohne SER-Schicht, d.h. auch die beleuchtete SER-Schicht wirkt noch biotoxisch. Allerdings ist ihre Biotoxizität gegenüber der unbeleuchteten SER-Schicht stark verringert, was an der Reduktion der optischen Dichte der Kultur um weitere 60 % auf der unbeleuchteten Schicht klar zu erkennen ist.

Noch deutlicher wird die Änderung der Biotoxizität der SER-Schicht an einer Untersuchung der Zellproliferation. Der BrdU (5-bromo-2'-deoxy-uridin)-Test ermöglicht die Quantifizierung der Zellproliferation von Zellkulturen (in unserem Fall Primärfibroblasten aus der 2. bis 4. Passsage von einem gesunden Donor) gezüchtet in Mikrotiterplatten über die Bestimmung des in der Zell-DNA eingebauten BrdU. Dem Zellgut wird BrdU-haltiges Medium zugegeben. Proliferierende Zellen bauen den BrdU-Zusatz an Stelle von Thymidin (Baustein der DNA) in ihre DNA ein. Nach Inkubationszeiten von 4 bis 24 Stunden wird das Medium entfernt, die Zellen gewaschen und fixiert. Dadurch erhält man einen leichteren Zugang zu dem BrdU, was von einem spezifischen Antikörper, welcher ein Markerenzym trägt (Peroxidase), erkannt wird. Es bilden sich markierte Antigen-Antikörper-Komplexe. Addiert man nun das Substrat des Markerenzyms zu diesen Komplexen hinzu, in unserem Fall ABTS, so findet eine Reaktion statt, in welcher das Substrat in die oxidierte Form überführt wird. Die oxidierte Form des ABTS liegt in gelöster Form vor und ist von grünblauer Farbe. Die optische Dichte der Lösungen lässt sich photometrisch bei 405 nm (Referenz: 490nm) messen. Somit lassen sich Rückschlüsse auf die Menge von eingebautem BrdU und damit auf die Proliferation ziehen.

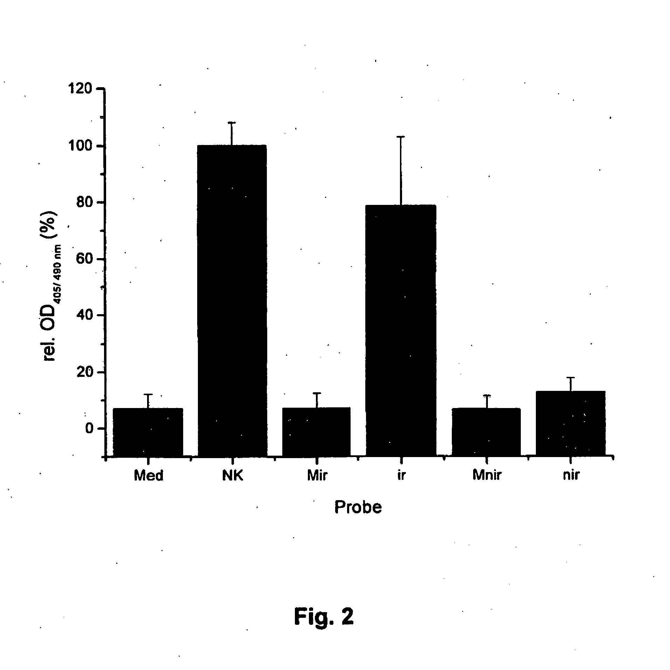

Gemessen wird das Verhältnis der optischen Dichten (rel. OD) von 405 nm und 490 nm als Maß für die Zellproliferation. Das Ergebnis ist als Histogramm in Fig. 2 gezeigt. Die Beschriftung der Histogramm-Säulen hat sinngemäß dieselbe Bedeutung wie in Fig. 1. Es sollte hier hervorgehoben werden, dass die Zellproliferation auf der beleuchteten SER-Schicht nur um ca. 20 % verringert wird, während sie auf der unbeleuchteten SER-Schicht annähernd völlig unterbunden wird.

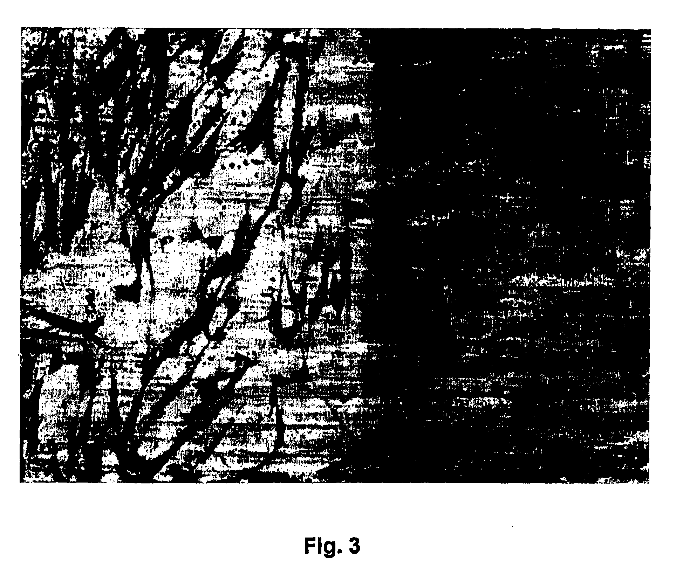

Abschließend wird noch mittels Lichtmikroskopie an gefärbten (Giemsa-Färbung) Präparaten die Zellmorphologie an der Grenze zwischen belichteter und unbelichteter SER-Schicht untersucht. Zu diesem Zweck werden 24 Stundenkulturen von primären Fibroblasten der 2. bis 4. Passage auf teilweise belichteten Oberflächen angelegt. Nach erfolgter Inkubation (in 5 % CO₂, bei 38 °C) werden die Zellen mit Glutaraldehyd fixiert und mit Giemsa gefärbt. Die sichtbaren Ergebnisse sind für maskiert belichtete Oberflächen (Belichtung mit UV(250-400 nm) für 20 Minuten vor Zellaussaat); links: belichtet, rechts: unbelichtet) in Fig. 3 zu sehen. Im belichteten Bereich ist ein deutlich dichteres Wachstum mit lang gestreckten, spindelförmigen Fibroblasten zu sehen, währen im unbelichteten Bereich nur einige verkürzte und tote Zellen zu verzeichnen sind.

## Patentansprüche

1. Verfahren zum Herstellen einer Implantatbeschichtung mit definierter Toxizität, **gekennzeichnet durch** die Schritte:
i. Herstellen eines Sols aus einer biotoxisch wirkenden Edelmetall-Ionen enthaltenden Lösung und einer Precursor-Lösung für ein Titanoxid,
ii. Beschichten eines Implantats **durch** Aufbringen des Sols mit einem Sol-Gel-Verfahren,
iii. Pyrolysieren und Sintern der Beschichtung unter Ausschluss von Licht, wobei der Edelmetall-Ionen-Anteil in der getrockneten Beschichtung zwischen 10 und 60 % der Gesamtmasse der getrockneten Beschichtung beträgt und
iv. Beleuchten von wenigstens Teilflächen der unter Lichtausschluss hergestellten Beschichtung zur Reduzierung der Toxizität der getrockneten Beschichtung auf ein vorbestimmtes Maß.

2. Verfahren nach Anspruch 1, **dadurch gekennzeichnet, dass** das Beleuchten unter gleichzeitigem Erwärmen wenigstens der beleuchteten Teilflächen erfolgt.

3. Verfahren nach einem der vorhergehenden Ansprüche, **dadurch gekennzeichnet, dass** das Beleuchten der unter Lichtausschluss hergestellten Beschichtung auf einer Mehrzahl von räumlich getrennten Teilflächen simultan erfolgt.

4. Verfahren nach einem der vorhergehenden Ansprüche, **dadurch gekennzeichnet, dass** die unter Lichtausschluss hergestellte Beschichtung vor dem Beleuchten mit einer lichtundurchlässigen Schablone, die Aussparungen aufweist, teilweise abgedeckt wird.

5. Verfahren nach einem der vorhergehenden Ansprüche, **dadurch gekennzeichnet, dass** zwischen dem Pyrolysieren und Sintern unter Ausschluss von Licht und dem Beleuchten der unter Lichtausschluss hergestellten Beschichtung eine Lagerzeit unter Lichtausschluss vorgesehen wird.

6. Verfahren nach einem der vorhergehenden Ansprüche, **dadurch gekennzeichnet, dass** das Beleuchten der unter Lichtausschluss hergestellten Beschichtung unter Erwärmen wenigstens der beleuchteten Teilflächen auf Temperaturen zwischen 80 °C und 250 °C erfolgt.

7. Verfahren nach einem der vorhergehenden Ansprüche, **dadurch gekennzeichnet, dass** als Edelmetall-Ionen Silberionen verwendet werden, wobei der Silber-Massenanteil bezogen auf die Masse der getrockneten Beschichtung zwischen 30 und 60 % eingerichtet wird.

8. Verfahren nach Anspruch 7, **dadurch gekennzeichnet, dass** die Silberionen enthaltende Lösung durch Zugabe von Pyridin stabilisiert wird.

9. Verfahren nach einem der vorhergehenden Ansprüche, **dadurch gekennzeichnet, dass** das Pyrolysieren unter Ausschluss von Licht bei Temperaturen um 250 °C und das Sintern unter Ausschluss von Licht bei Temperaturen zwischen 450 °C und 550 °C erfolgt.

10. Implantat mit einer Silberionen enthaltenden Titanoxidbeschichtung, wobei die Silberionen durch thermische Sinterung eines Precursor-Materials in die Beschichtung eingebettet sind,
**dadurch gekennzeichnet, dass**
der Silberionenanteil in der getrockneten Beschichtung zwischen 30 und 60 % der Gesamtmasse der getrockneten Beschichtung beträgt, und
die Silberionen durch Lichteinstrahlung reduzierbar sind.

11. Implantat nach Anspruch 10, **dadurch gekennzeichnet, dass** die Silberionen durch UV-Licht reduzierbar sind.

## Claims

1. A method of producing an implant coating with a defined toxicity, **characterised by** the steps of:
i. producing a sol from a biotoxically acting solution that contains precious metal ions and a precursor solution for a titanium oxide,
ii. coating an implant by applying the sol with a sol-gel process,
iii. pyrolysing and sintering the coating with the exclusion of light, the proportion of the precious metal ions in the dried coating being between 10 and 60% of the total mass of the dried coating, and
iv. irradiating at least partial areas of the coating produced with the exclusion of light to reduce the toxicity of the dried coating to a predetermined degree.

2. The method according to claim 1, **characterised in that** the irradiation takes place while simultaneously heating at least the irradiated partial areas.

3. The method according to one of the preceding claims, **characterised in that** the irradiation of the coating produced with the exclusion of light is carried out simultaneously on a plurality of spatially separate partial areas.

4. The method according to one of the preceding claims, **characterised in that** the coating produced with the exclusion of light is partially covered with a light-impermeable template having openings before it is irradiated.

5. The method according to one of the preceding claims, **characterised in that**, between the pyrolysing and sintering with the exclusion of light and the irradiation of the coating that has been produced with the exclusion of light, a storage time with the exclusion of light is provided.

6. The method according to one of the preceding claims, **characterised in that** the irradiation of the coating that has been produced with the exclusion of light is carried out while heating at least the irradiated partial areas to temperatures of between 80°C and 250°C.

7. The method according to one of the preceding claims, **characterised in that** silver ions are used as the precious metal ions, the proportion of silver by mass, based on the mass of the dried coating, being adjusted to between 30 and 60%.

8. The method according to claim 7, **characterised in that** the solution containing silver ions is stabilised by adding pyridine.

9. The method according to one of the preceding claims, **characterised in that** the pyrolysing is performed at temperatures of about 250°C with the exclusion of light and the sintering is performed at temperatures of between 450°C and 550°C with the exclusion of light.

10. An implant with a titanium oxide coating containing silver ions, wherein the silver ions are embedded into the coating by thermal sintering of a precursor material,
**characterised in that**
the proportion of silver ions in the dried coating is between 30 and 60% of the total mass of the dried coating and
the silver ions can be reduced by light irradiation.

11. The implant according to claim 10, **characterised in that** the silver ions can be reduced by UV light.

## Revendications

1. Procédé pour la fabrication d'un revêtement d'implant présentant une toxicité définie, **caractérisé par** les étapes suivantes :
i. fabrication d'un sol à partir d'une solution ayant une action biotoxique comprenant des ions de métaux nobles et d'une solution précurseur pour un oxyde de titane,
ii. enduction d'un implant par application du sol à l'aide d'un procédé sol-gel,
iii. pyrolyse et frittage de l'enduction à l'abri de la lumière, la teneur en ions de métaux nobles dans le revêtement sec étant comprise entre 10 et 60 % de la masse totale du revêtement sec, et
iv. éclairage au moins de surfaces partielles du revêtement fabriqué à l'abri de la lumière en vue de réduire la toxicité du revêtement sec à une valeur prédéterminée.

2. Procédé selon la revendication 1, **caractérisé en ce que** l'éclairage est réalisé tout en chauffant simultanément au moins les surfaces partielles éclairées.

3. Procédé selon l'une des revendications précédentes, **caractérisé en ce que** l'éclairage du revêtement fabriqué à l'abri de la lumière est réalisé sur une pluralité de surfaces partielles séparées dans l'espace.

4. Procédé selon l'une des revendications précédentes, **caractérisé en ce que** le revêtement fabriqué à l'abri de la lumière est recouvert partiellement d'un pochoir opaque à la lumière et qui présente des évidements.

5. Procédé selon l'une des revendications précédentes, **caractérisé en ce qu'**une période de stockage à l'abri de la lumière est prévue entre la pyrolyse et le frittage à l'abri de la lumière et l'éclairage du revêtement fabriqué à l'abri de la lumière.

6. Procédé selon l'une des revendications précédentes, **caractérisé en ce que** l'éclairage du revêtement fabriqué à l'abri de la lumière est réalisé en chauffant au moins les surfaces partielles éclairées à des températures comprises entre 80 °C et 250 °C.

7. Procédé selon l'une des revendications précédentes, **caractérisé en ce que** des ions argent sont utilisés comme ions de métaux nobles, la teneur massique en argent rapportée à la masse du revêtement sec est comprise entre 30 et 60 %.

8. Procédé selon la revendication 7, **caractérisé en ce que** la solution contenant les ions argent est stabilisée par ajout de pyridine.

9. Procédé selon l'une des revendications précédentes, **caractérisé en ce que** la pyrolyse est réalisée à l'abri de la lumière à des températures d'environ 250 °C et le frittage est réalisé à l'abri de la lumière à des températures comprises entre 450 °C et 550 °C.

10. Implant avec un revêtement d'oxyde de titane contenant des ions argent, les ions argent étant enrobés dans le revêtement par frittage thermique d'un matériau précurseur,
**caractérisé en ce que**
la teneur en ions argent dans le revêtement sec est comprise entre 30 et 60 % de la masse totale du revêtement sec, et les ions argents peuvent être réduits par rayonnements lumineux.

11. Implant selon la revendication 10, **caractérisé en ce que** les ions argent peuvent être réduits par de la lumière UV.
